# EUROPÄISCHE PATENTSCHRIFT

(11) **EP 1 706 197 B1**
(45) Veröffentlichungstag und Bekanntmachung des Hinweises auf die Patenterteilung: **28.11.2007**
(21) Anmeldenummer: 05700709.8
(22) Anmeldetag: 05.01.2005
(51) Int. Cl.: B01D 71/02, B01J 20/18, B01D 53/22

(54) **KOMPOSIT-MEMBRAN**
COMPOSITE MEMBRANE
MEMBRANE COMPOSITE

(30) Priorität: 13.01.2004 DE 102004001974
(43) Veröffentlichungstag der Anmeldung: 04.10.2006
(73) Patentinhaber: BASF Aktiengesellschaft, 67056 Ludwigshafen (DE)
(72) Erfinder: BITTERLICH, Stefan, 67246 Dirmstein (DE); VOSS, Hartwig, 67227 Frankenthal (DE); SCHUCH, Gunter, 67071 Ludwigshafen (DE); DIEFENBACHER, Armin, 76726 Germersheim (DE); NOACK, Manfred, 12683 Berlin (DE); SCHÄFER, Roland, 73430 Aalen (DE); VOIGT, Ingolf, 07743 Jena (DE); RICHTER, Hannes, 07629 Hermsdorf (DE); CARO, Jürgen, 13129 Berlin (DE)
(74) Vertreter: Isenbruck, Günter
(86) Internationale Anmeldenummer: PCT/EP2005/000047
(87) Internationale Veröffentlichungsnummer: WO 2005/068057

(56) Entgegenhaltungen:
- EP-A- 0 460 512
- GB-A- 2 340 112

## Beschreibung

Die vorliegende Erfindung betrifft eine Komposit-Membran, die mindestens einen porösen Träger und mindestens eine mikroporöse Trennschicht enthält, wobei die Trennschicht einen Zeolithen vom MFI-Typ enthält. Weiterer Gegenstand der vorliegenden Erfindung ist ein Verfahren zur Herstellung dieser Komposit-Membranen sowie ein Verfahren zur Trennung von Olefinen, in dem die erfindungsgemäßen Komposit-Membranen verwendet werden.

In chemischen und petrochemischen Produktionsverfahren stellt sich häufig die Aufgabe der Auftrennung von Olefinen enthaltenden Stoffgemischen. Im Allgemeinen kann diese Auftrennung durch destillative Verfahren ausreichend gut gelöst werden. Allerdings ist im Fall sehr engsiedender Gemische eine wirtschaftlich durchführbare destillative Aufarbeitung nicht oder nur unter Einsatz von Hilfsstoffen, welche zusätzliche Verfahrensschritte notwendig machen, möglich. Ein Beispiel für die Auftrennung von eng siedenden Gemischen ist die Trennung isomerer Olefine mit gleicher Kohlenstoffanzahl.

In der Literatur sind Arbeiten zur Trennung derartiger Gemische mittels eines Membranverfahrens beschrieben, wobei die Verfahren im allgemeinen so durchgeführt werden, dass ein zu trennender Stoffstrom (Feed) in einer selektiv wirkenden Membran aufgetrennt und in ein Permeat, d. h. einen die Membran durchdringenden Stoffstrom, und ein Retentat, d.h. einen die Membran nicht durchdringenden Stoffstrom, aufgetrennt wird.

Eine Trennung linearer und verzweigter Kohlenwasserstoffisomere mittels organischer Membranen, die aufgrund des unterschiedlichen sterischen Anspruchs der isomeren Molekühle erfolgt, ist grundsätzlich möglich und wird beispielsweise in NEFTEKHIMIYA V23 N.4, Seiten 435 bis 453 beschrieben. Die in diesem Verfahren verwendeten organischen Membranen weisen jedoch den Nachteil einer stark eingeschränkten thermischen und chemischen Stabilität auf.

EP 0 180 200 A beschreibt die selektive Abtrennung von n-Paraffinen und n-Olefinen aus einem Naphtha-Destillat über eine keramische Membran, die aus CaA-Zeolithen, enthalten in den Poren eines porösen Al₂O₃-Rohres, besteht.

In Proc. 6th. Int. Zeolite Conference, Reno, USA, 1983, Eds. D. Olson, A. Bisio, Butterworth, London, 1984, Seiten 217 bis 224 wird die Trennung von n- und iso-Butan mittels einer Membran aus Einkristallen des Zeolithen Silikalith, eingebettet in eine Polymermatrix, beschrieben.

In US 4,699,892 wird die Abtrennung von 1-Buten aus einem C₄-Kohlenwasserstoff über eine CaA-Membran beschrieben.

WO 94/25151 und WO 94/01209 beschreiben die Herstellung von Komposit-Membranen, d.h. Membranen, bei denen die eigentliche trennaktive Schicht aus einem Zeolith vom MFI-Typ auf einem makroporösen Träger aufgebracht ist, durch eine in situ-Hydrothermalsynthese. Die hieraus resultierenden Membranen, die zur Trennung von n-Butan/Isobutan-Mischungen verwendet werden, sind rein keramische und zeichnen sich durch eine hohe chemische und thermische Stabilität aus. Allerdings weisen sie im Allgemeinen auch eine hohe katalytische Aktivität auf, die im Falle von Olefinen enthaltenden Mischungen meist unerwünscht ist, weil die katalytische Aktivität dabei zum Verlust an wertvollen Komponenten der zu trennenden Mischung bzw. zur Bildung von unerwünschten Reaktionsprodukten führt. So können sich beispielsweise im Falle von 1-Buten und/oder Isobuten enthaltenden Mischungen, wie allgemein bekannt, an in Zeolithen enthaltenen reaktiven Zentren aus 1-Buten durch Isomerisierung 2-Butene und aus Isobuten dimere und oligomere Produkte bilden.

US 5,019,263 lehrt, dass bei einer zeolithischen Nicht-Komposit-Membran, d. h. einer Membran, die allein aus der zeolithischen Trennschicht besteht, die katalytische Aktivität durch Einbringen von Alkali- oder Erdalkaliionen in die Membran verringert werden kann. Nicht-Komposit-Membrane weisen jedoch den Nachteil auf, dass die Trennschicht, um eine für technische Anwendung ausreichend mechanische Stabilität sicher zu stellen, eine erhebliche Dicke aufweisen muss, was dazu führt, dass unter gegebenen Bedingungen die transmembrane Flussdichte deutlich geringer ist als im Fall einer Komposit-Membran, bei der die Dicke der Trennschicht typischerweise im Bereich einiger µm liegt.

Aufgabe der vorliegenden Erfindung ist es somit, Komposit-Membranen bereitzustellen, die im Wesentlichen keine katalytische Aktivität und eine ausreichend hohe mechanische Stabilität aufweisen.

Erfindungsgemäß wird diese Aufgabe gelöst durch Komposit-Membranen, umfassend mindestens einen porösen Träger und mindestens eine mikroporöse Trennschicht, die mindestens einen Zeolith vom MFI-Typ enthalten.

Die erfindungsgemäßen Komposit-Membranen sind dadurch gekennzeichnet, dass die Trennschicht durch eine Hydrothermalsynthese hergestellt wird, bei der das Molverhältnis von Silizium zu Aluminium in der Syntheselösung größer 120, vorzugsweise größer 200, besonders bevorzugt größer 300, ist und der Träger in einer an die Trennschicht angrenzenden Zone von mindestens 100 nm, besonders bevorzugt mindestens 250 nm, insbesondere mindestens 500 nm, weniger als 10 Gew.-%, besonders bevorzugt weniger als 5 Gew.-%, insbesondere weniger 1 Gew.-%, Aluminium in elementarer oder chemisch gebundener Form enthält und das Molverhältnis von Silizium zu Aluminium in der Trennschicht größer 120, besonders bevorzugt größer 200, insbesondere größer 300, ist.

Geeignete Träger für die erfindungsgemäßen Komposit-Membranen sind Körper mit durchgängigen Poren mit Porendurchmessern zwischen 0,5 nm und 3 µm, besonders bevorzugt 1 nm bis 60 nm, insbesondere 5 nm bis 60 nm. Diese Körper können beispielsweise die Form von flachen Scheiben, Rohren oder Kapillaren aufweisen.

Vorteilhaft ist auch die Verwendung von so genannten Multikanalelementen als Träger, wie sie bei keramischen Membranen für die Mikro- oder Ultrafiltration Anwendung finden.

Unabhängig von der zuvor geschilderten geometrischen Form des Trägers ist ein so genannter asymmetrischer Aufbau des Trägermaterials vorteilhaft, bei dem dieser aus mehreren aufeinander folgenden Schichten mit abnehmendem Porendurchmesser besteht, wobei sich auf der mit der Trennschicht zu versehenden Seite des Trägers der kleinste Porendurchmesser befindet.

Der kleinste Porendurchmesser des Trägers beträgt vorzugsweise 0,5 bis 100 nm, besonders bevorzugt 0,75 bis 80 nm, insbesondere 1 bis 60 nm.

Als Material für den Träger eignet sich eine Vielzahl von Materialien, wie beispielsweise Sintermetalle, Stähle oder oxidkeramischen Materialien, wie z. B. Aluminiumoxid, Titandioxid oder überwiegend aus Titandioxid bestehende Mischungen von Metalloxiden. Ferner sind Siliziumdioxid, Zirkoniumdioxid, Magnesiumoxid oder andere Metalloxide, sofern sie eine geringe Löslichkeit unter hydrothermalen Bedingungen in der Syntheselösung aufweisen, d.h. bei einem pH-Wert von 11 bis 12, einer Temperatur von 170 bis 190 °C, einem Druck von 10 bis 16 bar und einer Konzentration von kleiner 1 mg/l, geeignet. Für die erfindungsgemäßen Membranen ist es wesentlich, dass das Molverhältnis von Silizium zu Aluminium in der Trennschicht größer 120, besonders bevorzugt größer 200, insbesondere größer 300, ist. Dieses Verhältnis bewirkt, dass in der Trennschicht der erfindungsgemäßen Membran die Anzahl an sauren bzw. ionischen Stellen gering ist. Dadurch wird eine Isomerisierung oder Nebenproduktbildung bei einer Stofftrennung von isomeren Olefinen vermindert oder sogar vermieden. Um den zuvor genannten Bereich des Silizium/Aluminium-Molverhältnisses zu erreichen ist es bevorzugt, dass als Trägermaterial ein möglichst aluminiumarmes Material verwendet wird. In einer bevorzugten Ausführungsform besteht somit der gesamte Träger aus einem oder mehreren Materialien, die aluminiumarm sind, d.h. weniger als 1 Gew.-%, besonders bevorzugt weniger als 0,1 Gew.-%, insbesondere weniger als 0,01 Gew.-%, Aluminium (im Sinne von elementarem oder chemisch gebundenem Aluminium) enthalten.

Bevorzugt ist der Träger an den Stellen, an denen die aus ihm hervorgehende Membran mit einem Dichtungsmaterial in Berührung kommt, mit einer geeigneten Hilfsschicht zu versehen, besonders bevorzugt mit einem an sich bekannten gasdichten und alkalibeständigen Glaslot.

In einer bevorzugten Ausführungsform der erfindungsgemäßen Komposit-Membranen ist die Trennschicht so beschaffen, dass bei einer Permeationsmessung mit einer äquimolaren 1-Buten/Isobuten-Mischung die bei einem Feeddruck von 1 bis 100 bar (a), besonders bevorzugt 2 bis 35 bar (a), insbesondere 5 bis 30 bar (a), einem unter dem Feeddruck liegenden Permeatdruck von 0,001 bis 10 bar (a), besonders bevorzugt 1 bis 10 bar (a), insbesondere 4 bis 8 bar (a), einer Temperatur zwischen 50 und 200 °C, besonders bevorzugt 50 und 150 °C, insbesondere 50 und 130 °C und einem Stufenschnitt (Permeatmenge/Feedmenge) von kleiner 0,1, besonders bevorzugt kleiner 0,05, insbesondere kleiner 0,01, die 1-Buten-Konzentration im Permeat größer 60 %, besonders bevorzugt größer 70 %, insbesondere größer 80 %, ist.

Die Trennschicht besteht vorzugsweise aus MFI-Zeolith, besonders bevorzugt Silikalith.

Weiterer Gegenstand der vorliegenden Erfindung ist ein Verfahren zur Herstellung der zuvor beschriebenen Komposit-Membranen, das durch die folgenden Verfahrensschritte gekennzeichnet ist:
(a) Hydrothermalsynthese einer Syntheselösung auf einem Träger durch in Kontaktbringen des Trägers mit der Syntheselösung über einen Zeitraum von 1 h bis 100 h, vorzugsweise 5 bis 50 h, besonders bevorzugt 10 bis 20 h, bei einer Temperatur von 100 bis 250 °C, vorzugsweise 140 bis 210 °C, besonders bevorzugt 170 bis 190 °C,
(b) Spülen der aus Verfahrensschritt (a) resultierenden Membran mit Wasser oder einer sauren Lösung für einen Zeitraum von 5 bis 120 min, vorzugsweise 10 bis 90 min,
(c) Trocknen der Membran bei einer Temperatur von 5 bis 40 °C, vorzugsweise 15 bis 35 °C, besonders bevorzugt 20 bis 30 °C, über einen Zeitraum von 1 bis 100 h, vorzugsweise 10 bis 30 h, besonders bevorzugt 10 bis 15 h, in Gegenwart eines strömenden oder ruhenden Gases,
(d) Kalzinierung der Membran mit einer Aufheizrate von 0,1 bis 1 K/min, vorzugsweise 0,1 bis 0,8 K/min, besonders bevorzugt 0,1 bis 0,75 K/min, bis zu einer Temperatur von 200 bis 600 °C, vorzugsweise 350 bis 500 °C, besonders bevorzugt 400 bis 500 °C, wobei bei der Endtemperatur 30 bis 500 min, vorzugsweise 300. bis 450 min, besonders bevorzugt 360 bis 400 min, verweilt wird und anschließend mit einer Rate von 0,1 K/min bis 10 K/min, vorzugsweise 0,5 bis 3 K/min, besonders bevorzugt 0,3 bis 1 K/min, abgekühlt wird.

In einer bevorzugten Ausführungsform der vorliegenden Erfindung werden die Verfahrensschritte in der Reihenfolge (a), (b), (c) und (d) durchgeführt.

In einer besonders bevorzugten Ausführungsform des erfindungsgemäßen Verfahrens wird das in Verfahrensschritt (a) eingesetzte Trägermaterial vor der Hydrothermalsynthese einem Seedingschritt unterzogen, bei dem auf der zu beschichtenden Seite des Trägers eine diese Seite zumindest teilweise bedeckende Schicht von Seedpartikeln aufgebracht wird, und danach erst die beschriebene Hydrothermalsynthese erfolgt.

Die Seedpartikel können vorzugsweise amorphe oder kristalline Körper mit Partikelgrößen zwischen 1 und 1000 nm, besonders bevorzugt 10 bis 100 nm, insbesondere 50 bis 80 nm, sein, die in ihrer chemischen Zusammensetzung im Wesentlichen der zu synthetisierenden Trennschicht entsprechen. In einer besonders bevorzugten Variante bestehen die Seedpartikel aus Silikalith.

Die Herstellung der Seedpartikel erfolgt durch einen separaten hydrothermalen Prozess. Die dabei verwendete Syntheselösung enthält vorzugsweise Siliziumdioxid, Natriumoxid, Tetrapropylammoniumhydroxid, Tetrapropylammoniumbromid, Ethanol und Wasser. Die Syntheselösung für die Herstellung der Seedpartikel enthält, jeweils unabhängig voneinander, vorzugsweise die zuvor genannten Komponenten in folgendem molaren Verhältnis:
- 1,0 Tetraethylorthosilkat TEOS
- 0 bis 0,01, besonders bevorzugt 0 bis 0,006, Natriumoxid Na₂O
- 0,2 bis 0,5, besonders bevorzugt 0,3 bis 0,5, insbesondere 0,3 bis 0,36, Tetrapropylammoniumhydroxid TPAOH
- 10 bis 20, besonders bevorzugt 12 bis 15, Wasser.

Tetrapropylammoniumhydroxid kann teilweise oder vollständig gegen Tetrapropylammoniumbromid ausgetauscht werden, wenn gleichzeitig eine equimolare Erhöhung des NaOH-Anteiles erfolgt.

Bei Verwendung von Tetraethylorthosilkat (TEOS) als Si-Quelle entstehen 4 mol Ethanol, dabei werden 2 mol Wasser pro mol Tetraethylorthosilkat (TEOS) verbraucht.

Die Seedsynthese erfolgt vorzugsweise weitgehend aluminiumfrei, d.h. die Syntheselösung für die Herstellung der Seeds ist durch ein Si/Al-Molverhältnis von vorzugsweise größer 120, besonders bevorzugt größer 200, insbesondere größer 300 gekennzeichnet.

Die genannten Tetrapropylammoniumsalze in der Syntheselösung zu dem Herstellen der Seedschicht dienen als strukturgebende Agenzien (Template). Es können allerdings auch andere Template außer Tetrapropylammoniumsalze verwendet werden, beispielsweise 1,6-Hexandiol und/oder Piperazin. Eine ausführliche Beschreibung findet sich hierzu in: R. Szostak, Handbook of Molecular Sieves, Seite 521.

Die Syntheselösung zur Herstellung der Seedpartikel wird vorzugsweise über einen Zeitraum von vorzugsweise 10 bis 500 h, besonders bevorzugt 50 bis 200 h , insbesondere 72 bis 120 h, vorzugsweise zwischen 60 und 100 °C, besonders bevorzugt zwischen 60 und 90 °C, langsam gerührt oder stehen gelassen. Die Weiterverarbeitung der Suspension erfolgt vorzugsweise durch Verdünnen mit entionisiertem Wasser oder durch Abzentrifugieren des Feststoffes, mehrfaches Waschen und anschließendes Redispergieren in entionisiertem Wasser.

Das entionisierte Wasser, das zum Redispergieren verwendet wird, ist vorzugsweise auf einen pH-Wert von 8 bis 12, besonders bevorzugt 8 bis 9, eingestellt. Hierzu kann beispielsweise Natronlauge oder Ammoniakwasser verwendet werden.

Die Aufbringung der Seedpartikel auf den Träger kann auf verschiedene Arten erfolgen. Zum einen kann dies durch Auffiltrieren, so genanntes slip casting, geschehen, d.h. eine die Seedpartikel enthaltende, vorzugsweise wässrige Lösung wird mit der zu beschichtenden Seite des Trägers in Kontakt gebracht und, entweder infolge von Anlegen eines Überdruckes auf der zu beschichtenden Seite gegenüber der nicht zu beschichtenden Seite, oder infolge eines von den Poren ausgeübten Kapillarsogs wird die die Seedpartikel umgebende Lösung in die Poren eingetragen, während die Seedpartikel sich, sofern sie größer als die Poren des Trägers sind, auf der zu beschichtenden Seite des Trägers akkumulieren. Zum andern kann das Anheften der Seedpartikel an den Träger auch durch ein geeignetes Hilfsmittel erfolgen. Beispielsweise eignen sich hierfür polymere quaternäre Ammoniumsalze, wie Poly-DADMAC (Redifloc®).

Der Verfahrensschritt (a) des erfindungsgemäßen Verfahrens umfasst die Hydrothermalsynthese einer Syntheselösung auf einem Trägermaterial, das gegebenenfalls wie oben beschrieben zur Erzeugung einer Seedschicht vorbehandelt wurde, durch Inkontaktbringen des Trägermaterials mit der Syntheselösung über einen Zeitraum von 1 bis 100 h, vorzugsweise 5 bis 50 h, besonders bevorzugt 10 bis 20 h, bei einer Temperatur von 100 bis 250 °C, vorzugsweise 140 bis 210 °C, besonders bevorzugt 170 bis 190°C.

Als Syntheselösung für die Hydrothermalsynthese werden dem Fachmann an sich bekannten Mischungen eingesetzt. Diese haben vorzugsweise folgende molare Zusammensetzung:
- 100 mol Siliziumdioxid,
- 5 x 10⁻⁵ bis 2 x 10⁻¹ mol, besonders bevorzugt 6 x 10⁻⁵ bis 2 x 10⁻¹ mol, Aluminiumoxid Al₂O₃,
- 0 bis 2 mol, Natriumoxid Na₂O,
- 4 bis 11 mol, Tetrapropylammoniumhydroxid,
- 0 bis 3 mol, Tetrapropylammoniumbromid,
- 2000 bis 5000 mol, besonders bevorzugt 2000 bis 3000 mol Wasser.

Bei Verwendung von 100 mol Tetraethylarthosilikat (TEOS) als Si-Quelle entstehen 400 mol Ethanol, es werden 200 mol Wasser verbraucht.

In dem erfindungsgemäßen Verfahren zur Herstellung der Membrane ist während der Hydrothermalsynthese das Molverhältnis zwischen Silizium und Aluminium vorzugsweise größer 120, besonders bevorzugt größer 200, insbesondere größer 300.

Die genannten Tetrapropylammoniumsalze dienen, ähnlich wie bei der Seedlösung, als strukturgebende Agenzien (Template). Es können stattdessen aber auch andere Template verwendet werden, beispielsweise 1,6-Hexandiol und Piperazin. Eine ausführliche Beschreibung hierzu findet sich in: R. Szostak, Handbook of Molecular Sieves, Seite 521.

Als Siliziumdioxidquelle wird vorzugsweise ein kolloidales Kieselsol wie Levasil der Firma Bayer oder eine siliziumorganische Verbindung wie z.B. Tetraethylorthosilikat (TEOS) verwendet. Als Al₂O₃-Quelle kann vorzugsweise das im genannten Silikat enthaltene Aluminium, eine Natriumaluminat-Lösung, metallische Aluminium, ein anorganisches Aluminiumsalz oder eine aluminiumorganische Verbindung, beispielsweise Aluminium-isopropylat, dienen. Das verwendete Wasser ist vorzugsweise ein mittels Ionentauscher entsalztes Wasser, besonders bevorzugt ein mittels Ionentauscher mit nachfolgender mindestens einmaliger Destillation entsalztes Wasser.

Die obige Syntheselösung wird vorzugsweise so hergestellt, dass Wasser, Tetrapropylammoniumhydroxid, Tetrapropylammoniumbromid und die Aluminiumquelle vorgemischt und für vorzugsweise 1 bis 120 min, besonders bevorzugt 5 bis 60 min, gerührt wird und anschließend vorzugsweise die Siliziumdioxidquelle in gelöster, kollodial gelöster oder suspendierter Form vorzugsweise innerhalb von 1 bis 100 min, besonders bevorzugt 2 bis 50 min, eingetragen wird.

Die Lösung wird vorzugsweise weitere 1 bis 200 min, besonders bevorzugt 5 bis 100 min, gerührt und 1 bis 150 min, besonders bevorzugt 5 bis 50 min, ohne Rühren gealtert.

Während der vorgenannten Schritte wird die Temperatur vorzugsweise bei 5 bis 100 °C, besonders bevorzugt 15 bis 40 °C, gehalten.

Die so hergestellte Syntheselösung wird anschließend für eine Dauer von 1 bis 100 h, besonders bevorzugt 5 bis 50 h, insbesondere 10 bis 20 h, mit dem gegebenenfalls geseedeten Träger in Kontakt gebracht. Dabei beträgt die Temperatur 100 bis 250° C, besonders bevorzugt 140 bis 210 °C, insbesondere 170 bis 190 °C.

Das Inkontaktbringen kann dabei auf verschiedene Weise erfolgen. So kann während der Synthesezeit die Syntheselösung im Wesentlichen ruhen oder sie kann stetig oder in regelmäßigen oder unregelmäßigen Abständen über den zu beschichtenden Träger in gleicher oder wechselnder Richtung bewegt werden. Vorteilhaft ist dabei eine Verfahrensweise, welche dafür sorgt, dass die Syntheselösung überwiegend mit den zu beschichtenden Oberflächen des Trägers in Berührung kommt und weniger mit der entgegengesetzten Seite. Ist beispielsweise im Falle einer Rohrmembran eine innenliegende Beschichtung erwünscht, so ist es vorteilhaft, den Kontakt der Syntheselösung mit der Rohraußenseite zu erschweren. Dieses kann zum einen erreicht werden, indem die nicht zu beschichtende(n) Oberfläche(n) in geeigneter Weise durch eine entfernbare und für die Syntheselösung schwer zu durchdringende Schicht abgedeckt werden. Im Falle des innenseitig zu beschichtenden Rohres kann diese abdeckende Schicht eine Umwicklung mit einem Band, beispielsweise aus PTFE sein oder eine geeignete aufstreichbare Polymerlösung. Zum anderen kann der Zutritt der Syntheselösung zu den nicht zu beschichtende(n) Oberfläche(n) aber auch dadurch erschwert werden, dass die Poren des Trägers während der Synthese mit einem Medium gefüllt sind, das den Durchtritt der Syntheselösung durch die Poren des Trägers erschwert bzw. verhindert. Bei diesem Medium kann es sich beispielsweise um eine Flüssigkeit, in der die Syntheselösung nur wenig löslich ist, handeln, oder einen Feststoff, der als Schmelze in die Poren des Trägers eingebracht wird und nach Beendigung der Synthese durch Schmelzen oder Lösen mit einem geeigneten Lösemittel entfernt wird, oder ein Gas, z.B. Luft oder Stickstoff, das sich in dem an die nicht zu beschichtende(n) Oberfläche(n) angrenzenden Raum und zumindest teilweise in den Poren des Trägers befindet, wobei der Druck des Gases so eingestellt wird, dass ein Durchtreten der Syntheselösung von der zu beschichtenden Seite auf die nicht zu beschichtende Seite des Trägers unterbunden wird.

In einer besonders bevorzugten Ausführungsform der vorliegenden Erfindung erfolgt dabei die Synthese der Trennschicht auf dem Trägermaterial so wie in der zeitgleich eingereichten deutschen Patentanmeldung DE 10 2004 001 975.4 mit dem Titel "Verfahren zur Herstellung von Membranen" der BASF AG (PF 0000055247/WW). In diesem Verfahren wird eine Membran hergestellt, die mindestens eine Feststoffschicht auf einer Seite eines porösen flächigen Trägers, der zu einer dreidimensionalen Struktur verformt sein kann enthält. Die Herstellung erfolgt durch Behandeln der zu beschichtenden Seite des Trägers mit einer die Feststoffschicht ausbildenden Syntheselösung. Bei der Herstellung der Feststoffschicht auf den porösen flächigen Träger wird der Raum, der sich hinter der nicht zu beschichtenden Seite des porösen Trägers befindet, mit einem inerten Fluid gefüllt, wobei der Druck und/oder die Temperatur des Fluids so gewählt werden, dass ein Kontakt der Syntheselösung mit der nicht zu beschichtenden Seite des porösen Trägers im wesentlichen verhindert wird. Insbesondere wird der Druck des Fluids während der Herstellung der Feststoffschicht auf einem Wert gehalten, der mindestens dem Druck der Syntheselösung entspricht, wenn der Kontaktwinkel zwischen Syntheselösung und Trägermaterial kleiner 90° ist. Ist der Kontaktwinkel zwischen Syntheselösung und Trägermaterial größer 90°, so wird der Druck während der Herstellung der Feststoffschicht insbesondere auf einem Wert gehalten, der höchstens dem Druck der Syntheselösung entspricht. Für weitere Ausführungen zu dieser Verfahrensweise wird auf die entsprechende DE-Anmeldung verwiesen, die durch Bezugnahme in die vorliegende Anmeldung eingeschlossen ist.

In einer besonderen Ausführungsform ist das erfindungsgemäße Verfahren somit dadurch gekennzeichnet, dass bei der Hydrothermalsynthese der Syntheselösung auf dem Träger der Zutritt der Syntheselösung zu der nicht zu beschichtenden Oberfläche oder den nicht zu beschichtenden Oberflächen des Trägers durch ein Medium im Wesentlichen verhindert wird, welches sich in den Poren des Trägers und auf der nicht zu beschichtenden Oberfläche oder den nicht zu beschichtenden Oberflächen befindet.

Es ist vorteilhaft, die Präparation der Seeds und der Syntheselösung sowie die Hydrothermalsynthese in Gefäßen durchzuführen, die praktisch kein Aluminium an die Lösungen abgeben kann. Geeignet sind hierfür aluminiumarme Stähle und/oder organische Polymere, beispielsweise PTFE, PFA, Polypropylen oder Werkstoffe, die auf den von der Lösung berührten Flächen mit mindestens einem der genannten Materialien beschichtet sind.

Nach der Synthese wird in dem Verfahrensschritt (b) zwecks Entfernung von Alkalispuren ein ein- oder mehrmaliges Spülen der erzeugten Membran mit Wasser oder einer sauren Lösung durchgeführt. Bei der sauren Lösung kann es sich um wässrige Lösungen von anorganischen oder organischen Säuren, beispielsweise Essig- oder Ameisensäure handeln, wobei die Säurekonzentration vorzugsweise 10⁻⁵ bis 1 mol/l, besonders bevorzugt 10⁻⁴ bis 0,01 mol/l, beträgt und die Dauer der Spülungen vorzugsweise 5 bis 120 min, besonders bevorzugt 10 bis 90 min, beträgt.

Danach erfolgt in Verfahrensschritt (c) eine Trocknung der Membran bei einer Temperatur von 5 bis 40 °C, besonders bevorzugt 20 bis 30 °C, für vorzugsweise 1 bis 100 h, besonders bevorzugt 10 bis 30 h, wobei sich über dem Trocknungsgut vorzugsweise ein strömendes oder ruhendes Gas, beispielsweise Stickstoff oder Luft, befindet.

In Verfahrensschritt (d) erfolgt eine Kalzinierung der Membran mit einer Aufheizrate von 0,1 bis 1 K/min, vorzugsweise 0,3 bis 0,7 K/min, bis zu einer Temperatur von 200 bis 600 °C, vorzugsweise 350 bis 500 °C, wobei bei der Endtemperatur 30 bis 500 min, vorzugsweise 400 bis 500 min, verweilt wird und anschließend mit einer Rate von 0,1 K/min bis 10 K/min, vorzugsweise 0,3 bis 1 K/min, abgekühlt wird. Während der Kalzinierung wird eine Zwischentemperatur von vorzugsweise 300 bis 450 °C, besonders bevorzugt von 380 bis 420 °C bei einer Haltezeit von vorzugsweise 100 bis 500 min, besonders bevorzugt 400 bis 450 min, aufrechterhalten.

An die Kalzinierung kann sich eine Nachbehandlung mit einer Protonen aufnehmenden Flüssigkeit anschließen. Bei der Protonen aufnehmenden Flüssigkeit handelt es sich bevorzugt um eine wässrige Lösung von einem oder mehreren Stoffen, die als Brönstedt-Basen wirken, beispielsweise Ammoniak, Alkalihydroxide, Alkalicarbonate oder, besonders bevorzugt, Alkalihydrogencarbonate. Die dabei verwendeten Lösungen sind vorzugsweise 0,001 bis 1 N, besonders bevorzugt 0,005 bis 0,1 N. Dieser Behandlungsschritt wird vorzugsweise für einen Zeitraum von 10 bis 200 min, besonders bevorzugt 30 bis 100 min, durchgeführt.

Die so erzeugte Membran kann noch einem weiteren Behandlungsschritt unterzogen werden, bei dem zwecks Schließung eventueller Defektstellen mindestens eine weitere Schicht aufgebracht wird, z.B. aus einem Metalloxid, vorzugsweise Siliciumdioxid, oder einem Polymer, vorzugsweise einem Polydialkylsiloxan, besonders bevorzugt Polydimethylsiloxan.

Weiterer Gegenstand der vorliegenden Erfindung sind die durch das erfindungsgemäße Verfahren herstellbaren Komposit-Membranen.

Weiterer Gegenstand der vorliegenden Erfindung ist ein Verfahren zur Trennung von Olefine enthaltenden Mischungen in mindestens einer Membranvorrichtung, enthaltend mindestens eine Membran, bei dem die Olefine enthaltende Mischung (Feed) in die Membranvorrichtung einströmt, mit mindestens einer Membran in Kontakt gebracht wird und in einen die Membran passierenden Strom (Permeat) und einen die Membran nicht passierenden Strom (Retentat) aufgetrennt wird.

Das erfindungsgemäße Trennverfahren ist dadurch gekennzeichnet, dass in dem Verfahren eine Komposit-Membran, wie sie zuvor beschrieben oder wie sie in dem erfindungsgemäßen Verfahren erhalten wird, verwendet wird.

Die Membranen kommen bei dem erfindungsgemäßen Verfahren zur Trennung von Olefinen vorzugsweise in Modulen zum Einsatz. In diesen Modulen wird jeweils eine der beschriebenen Membranen so eingedichtet, dass die MFI-Schicht den Feed- und den Permeatraum voneinander trennt. Die Eindichtung kann, sofern die Membranen in Form von Rohren oder Multikanalelementen vorliegen, mittels O-Ringen aus Elastomeren Viton® oder Kalrez® oder durch Eingießen der Elemente in eine polymere oder keramische Vergussmasse an mindestens einem Ende der Elemente und nachfolgendes Abschneiden der Vergussmasse erfolgen. Das Eingießen von lediglich einem Ende ist nur sinnvoll im Falle von Rohrmodulen, bei denen der Feedraum sich auf der Rohraußenseite befindet und bei denen die Rohre am nicht eingedichteten Ende verschlossen sind. Im Falle von Rohrmembranen oder Mehrkanalelementen weist der Mantelraum um die Rohre bevorzugt eine zylindrische Form auf.

In dem erfindungsgemäßen Verfahren zur Trennung von Olefinen sind vorzgusweise eines oder mehrere der beschriebenen Module Bestandteil(e) einer Membraneinheit. Dieses kann auf mehrere dem Fachmann an sich bekannte Arten betrieben werden. Beispiel hierfür ist die Gasseparation, bei der der Feedstrom gasförmig mit der Membran in Kontakt gebracht wird. Alternativ ist die Pervaporation denkbar, wobei das zu trennende Gemisch (Feed) in flüssiger Form mit der Membran in Kontakt gebracht und der die Membran passierende Strom (Permeat) gasförmig abgezogen wird.

Die Temperatur, bei dem das zu trennende Gemisch mit der Membran in Kontakt gebracht wird, liegt vorzugsweise zwischen 20 und 200 °C, besonders bevorzugt 50 bis 150 °C.

Der Druck beträgt auf der Feedseite der Membran vorzugsweise 1 bis 100 bar(a), besonders bevorzugt 1 bis 35 bar(a).

Der Druck beträgt auf der Permeatseite vorzugsweise 0,01 bis 10, besonders bevorzugt 1 bis 6 bar(a), wobei der Druck auf der Retentatseite der Membran im Allgemeinen höher als auf der der Permeatseite ist.

Der permeatseitige Druck wird vorzugsweise eingestellt durch Abführen des Permeatstroms mittels einer Vakuumpumpe und/oder eines Kompressors und/oder durch Kondensieren des Permeatstroms bei einer Temperatur, die zu einem dem gewünschten Permeatdruck entsprechenden Eigendruck des Permeatgemischs führt.

Es ist auch möglich, den Partialdruck der permeierenden Komponenten durch Einführen eines Spülgases (Sweepgas) auf die Permeatseite zu senken. Als Sweepgase sind beispielsweise Stickstoff oder Wasserdampf geeignet.

Im Falle der Pervaporation kann es vorteilhaft sein, die benötigte Membranfläche auf mehrere Vorrichtungen aufzuteilen und, zwecks Ausgleichs des durch den Phasenübergang flüssig-gasförmig verursachten Wärmeverlustes, einen oder mehrere Wärmeüberträger zwischen die Membranvorrichtungen zu schalten.

Das Membranverfahren kann gegebenenfalls einstufig ausgeführt werden, d. h. sowohl das Retentat als auch das Permeat aus einer Membranvorrichtung oder die vereinigten Permeate aus mehreren vom Feed hintereinander und/oder parallel durchströmten Membranvorrichtungen verlassen ohne weitere Behandlung die Membraneinheit. Das Membranverfahren kann aber auch zwei- oder mehrstufig durchgeführt werden, wobei aus einer Stufe das Permeat als Feed in die jeweils folgende Stufe geführt wird und das Retentat aus dieser Stufe dem Feed in die erstgenannte Stufe zugemischt wird.

Derartige Anordnungen sind an sich bekannt, siehe Sep. Sci. Technol. 31 (1996), Seite 729 ff.

Das genannte Verfahren ist insbesondere zur Trennung von Olefine enthaltenden Mischungen geeignet, bevorzugt für Trennungen von Gemischen linearer und verzweigter Olefine wie z.B. 1-Buten/Isobuten oder anderer Mischungen isomerer Butene. Es kann insbesondere dann vorteilhaft angewendet werden, wenn es zwecks Aufarbeitung eines Olefingemischs einem Verfahren vorgeschaltet ist, bei dem entweder ein lineares Olefin wie z.B. 1-Buten oder ein verzeigtes Olefin wie z.B. Isobuten benötigt wird.

Die vorliegende Erfindung wird anhand der nachfolgenden Beispiele näher erläutert.

### Ausführungsbeispiele:

### a) Vorbehandlung der Träger:

Drei poröse Träger in Rohrform (Länge 250 mm, Außendurchmesser 10 mm, Innendurchmesser 7 mm, Porengröße innen 5 nm für Membran 2 und 3 und 1 nm für Membran 1, Material siehe Tabelle, an den Enden versehen mit Glaslot) wurden zunächst mit Seeds (Silikalith-Kristalle mit einer Größe von 30 bis 100 nm) innen mittels Slip-Casting beschichtet. Danach wurde das Rohr mit einer Rate von 0,75 K/h auf 400 °C aufgeheizt, 7 h bei 400 °C gehalten und dann mit einer Rate von 0,75 K/h auf Raumtemperatur abgekühlt. Danach wurde das Rohr außenseitig mit PTFE-Band umwickelt und in eine mit gemäß nachfolgender Beschreibung hergestellte Syntheselösung gestellt.

### b) Herstellung der Syntheselösung:

Die Zusammensetzung der Syntheselösungen ist in der Tabelle angegeben. Die Quelle für SiO₂, Al₂O₃ und Na₂O war das Kieselsol Levasil® 300/30 % (Fa. Kurt Obermeier, Bad Berleburg, Deutschland) mit einem Verhältnis SiO₂/Al₂O₃/Na₂O von 90/0,15/1,66. Im Fall von Membran 1 wurde der Al₂O₃-Anteil darüber hinaus durch Zugabe von Aluminiumisopropylat (Fa. Strem Chemicals) angehoben, das auch die Quelle für C₃H₇OH darstellt.

Wasser (gereinigt über Ionentausch und zweifache Destillation), TPAOH (40 %ige wässrige Lösung, Fa. Alfa Aesar), TPABr (Fa. Merck) und, im Fall von Membran 1 Aluminiumisopropylat wurden in einen Erlenmeyerkolben aus Polypropylen gegeben und bei Raumtemperatur 30 min gerührt. Anschließend wurde das Levasil unter Rühren zugetropft, danach erfolgte eine Alterung bei Raumtemperatur in Ruhe über 30 min.

### c) Hydrothermalsynthese:

Die Synthesen erfolgten bei einer Temperatur von 180 °C über eine Dauer von 16 h, indem der kalte Autoklav mit der Syntheselösung in einen vorgeheizten Trockenschrank gestellt wurde. Nach der Synthese wurde das Teflonband entfernt.

### d) Nachbehandlung:

Die Membran wurde in einen Messzylinder gestellt und unter Rühren mit der Reinigungslösung (siehe Tabelle) mehrmals gewaschen. Anschließend wurden die Membranen ca. 20 h an der Raumluft trocknen gelassen und danach in einen Umluftofen gegeben, dort mit einer Rate von ca. 0,7 K/h zunächst auf 400 °C und nach einer Haltezeit von 400 min mit einer Heizrate von 0,1 K/min auf 450 °C aufgeheizt und dort für 400 min belassen. Anschließend wurde mit einer Rate von 0,75 K/h auf Raumtemperatur abgekühlt.

### e) Permeationsversuche:

Für die Permeationsversuche wurden die Membranen in einem Testmodul platziert. Die Abdichtung des Feedraums gegen den Permeatraum wurde mittels einer O-RingDichtung erreicht. Dabei wurden die O-Ringe auf die verglasten Enden des Trägers gesteckt. Das Testmodul wurde in einem Ofen platziert.

Vor den Messungen wurden die Membranen evakuiert und die Zuführungsleitungen und der Ofen auf 130 °C vorgeheizt. Danach wurden die Einzelgasflüsse von H₂, N₂, 1-Buten und i-Buten bestimmt. Nach jeder Einzelgasmessung wurde der Feed- und Permeatraum evakuiert. Das Testgas war eine 50/50-Mischung 1 Buten/Isobuten (Fa. Linde, Reinheit der Gase jeweils 99,5 %), vorliegend in einem Gaszylinder. Aus diesem wurde das Testmodul feedseitig angeströmt. Nach dem Testmodul wurde der feedseitige Druck mittels eines Hinterdruckreglers auf 2,5 bar (a) eingeregelt. Die Messung der Permeat- und Retentatmenge erfolgte über kommerziell erhältliche Seifenblasenzähler. Während der Permeationsmessungen wurde die Temperatur des Testmoduls bei 130 °C gehalten.

Der das Testmodul verlassende Permeatstrom (Permeatdruck: ca. 1 bar (a)) wurde in die Probennahme-Schleife eines GC-MS-Geräts geleitet und dort analysiert.

Außer den Komponenten des Testgemisches wurden zum einen die Isomerisierungsprodukte cis- und trans-2-Buten und zum anderen mehrere C₈-Kohlenwasserstoffe gefunden. Im Testgas selbst wurden keine C₈-Kohlenwasserstoffe gefunden. Die Konzentrtionen der 2-Butene im Testgas waren < 0,1 %. Die quantitative Bestimmung erfolgte für die isomeren Butene durch Vergleich der jeweiligen Ionenströme mit dem Signal des 1-Butens, für die C₈-Kohlenwasserstoffe durch Zumischen von trans-2-Octen als internem Standard.

Die Ergebnisse der Messungen sind zusammen mit den maßgeblichen Eigenschaften der hergestellten und untersuchten Membranen in der Tabelle dargestellt:

| | Membran 1 | Membran 2 | Membran 3 |
|---|---|---|---|
| Material des Trägers | TiO₂ 1 nm | Al₂O₃ | TiO₂ |
| | (innerste Schicht) auf Al₂O₃ | 5 nm | 5 nm |
| Molverhältnis in der Syntheselösung ¹⁾ | 90/0, 41/1, | 90/0, 15/1, | wie Membran 2 |
| | 66/3, 7/2, 3/1, | 66/3, 7/2, | |
| | 56/1990 | 3/0/1990 | |
| Waschung nach Hydrothermalsynthese | 4 x 1 h | 4 x 1 h verd. | wie Membran 2 |
| | Wasser | AS²⁾ | |
| Permeatkonzentrationen | | | |
| (Gewichtsanteile): | | | |
| 1-Buten im Permeat | 79 % | 85,6 % | 85 % |
| 2-Butene im Permeat | 2 % | 0,4 % | < 0,1 % |
| C₈-Kohlenwasserstoffe im Permeat | 0,5 % | 0,1 % | 17,5 ppb |
| ¹⁾ SiO₂/Al₂O₃/Na₂O/TPAOH/TPABr/C₃H₇OH/H₂O | | ²⁾ Ameisensäure | |
| | | 0,001 mol/l | |

Aus den Ergebnissen ist ersichtlich, dass die Erhöhung des Si/Al-Molverhältnisses bereits zu einer Verringerung der katalytischen Aktivität der Membran im Sinne der Olefin-lsomerisierung und -Dimerisierung führt, dass dieser Effekt aber wesentlich ausgeprägter ist bei der erfindungsgemäßen Kombination von hohem Si/Al-Molverhältnis und aluminiumarmem Träger.

## Patentansprüche

1. Komposit-Membran, bestehend aus einem porösen Träger und einer porösen Trennschicht, die aus einem Zeolith vom MFI-Typ besteht, **dadurch gekennzeichnet, dass** die Trennschicht durch eine Hydrothermalsynthese hergestellt wird, bei der das Molverhältnis von Silizium zu Aluminium in der Syntheselösung größer 120 ist und der Träger in einer an die Trennschicht angrenzenden Zone von mindestens 100 nm weniger als 10 Gew.-% Aluminium in elementarer oder chemisch gebundener Form enthält und dass das Molverhältnis von Silizium zu Aluminium in der Trennschicht größer 120 ist und das Trägermaterial asymmetrisch aufgebaut ist.

2. Komposit-Membran nach Anspruch 1, **dadurch gekennzeichnet, dass** der Träger ein Körper mit durchgängigen Poren mit einem Porendurchmesser von 5 nm bis 3 µm ist.

3. Komposit-Membran nach Anspruch 1 oder 2, **dadurch gekennzeichnet, dass** der Träger ausgewählt ist aus der Gruppe, bestehend aus Stählen, Sintermetallen, oxidkeramischen Materialien und Metalloxiden.

4. Verfahren zur Herstellung einer Komposit-Membran, die mindestens einen porösen Träger und mindestens eine poröse Trennschicht umfasst, wobei die Trennschicht mindestens einen Zeolith vom MFI-Typ enthält und das Molverhältnis von Silizium zu Aluminium in der Trennschicht größer 120 ist und wobei der Träger in einer an die Trennschicht angrenzenden Zone von mindestens 100 nm weniger als 10 Gew.-% Aluminium in elementarer oder chemisch gebundener Form enthält und das Trägermaterial asymmetrisch aufgebaut ist, **gekennzeichnet durch** die folgenden Verfahrensschritte:
(a) Hydrothermalsynthese einer Syntheselösung, bei der das Molverhältnis von Silizium zu Aluminium größer 120 ist, auf einem Träger **durch** Inkontaktbringen des Trägers mit der Syntheselösung über einen Zeitraum von 1 bis 100 h bei einer Temperatur von 100 bis 250 °C,
(b) Spülen der aus Verfahrensschritt (a) resultierenden Membran mit Wasser oder einer sauren Lösung für einen Zeitraum von 5 bis 120 min,
(c) Trocknen der Membran bei einer Temperatur von 5 bis 40 °C über einen Zeitraum von 1 bis 100 h in Gegenwart eines strömenden oder ruhenden Gases,
(d) Kalzinierung der Membran mit einer Aufheizrate von 0,1 bis 1 K/min bis zu einer Temperatur von 200 bis 600 °C, wobei bei der Endtemperatur 30 bis 500 min verweilt wird und anschließend mit einer Rate von 0,1 K/min bis 10 K/min abgekühlt wird.

5. Verfahren nach Anspruch 4, **dadurch gekennzeichnet, dass** das in Verfahrensschritt (a) eingesetzte Trägermaterial vor der Hydrothermalsynthese einem Seedingschritt unterzogen wird, bei dem auf der zu beschichtenden Seite des Trägers eine diese Seite zumindest teilweise bedeckende Schicht von Seedpartikeln aufgebracht

6. Verfahren nach Anspruch 4 oder 5, **dadurch gekennzeichnet, dass** bei der Hydrothermalsynthese der Syntheselösung auf dem Träger der Zutritt der Syntheselösung zu der nicht zu beschichtenden Oberfläche oder den nicht zu beschichtenden Oberflächen des Trägers durch ein Medium im Wesentlichen verhindert wird, welches sich in den Poren des Trägers und auf der nicht zu beschichtenden Oberfläche oder den nicht zu beschichtenden Oberflächen befindet.

7. Verfahren zur Trennung von Olefine enthaltenden Mischungen in mindestens einer Membranvorrichtung, enthaltend mindestens eine Membran, bei dem die Olefine enthaltende Mischung in die Membranvorrichtung einströmt, mit der mindestens einen Membran in Kontakt gebracht wird und in einen die Membran passierenden Strom und einen die Membran nicht passierenden Strom aufgetrennt wird, **dadurch gekennzeichnet, dass** in dem Verfahren eine Komposit-Membran gemäß einem der Ansprüche 1 bis 3 oder erhältlich nach dem Verfahren gemäß einem der Ansprüche 4 bis 6 verwendet wird.

8. Verfahren nach Anspruch 7, **dadurch gekennzeichnet, dass** die Auftrennung bei einer Temperatur von 20 bis 200 °C durchgeführt wird.

9. Verfahren nach Anspruch 7 oder 8, **dadurch gekennzeichnet, dass** bei der Auftrennung der Olefine enthaltenden Mischung der Druck auf der Permeatseite der Membran, 0,01 bis 10 bar (a) beträgt und/oder der Druck auf der Retentatseite der Membran höher ist als auf der Permeatseite.

10. Verwendung der Komposit-Membran nach einem der Ansprüche 1 bis 3 oder erhältlich nach dem Verfahren gemäß einem der Ansprüche 4 bis 6 zur Trennung von Olefine enthaltenden Mischungen.

11. Verwendung nach Anspruch 10, **dadurch gekennzeichnet, dass** die Mischung isomere Butene enthält.

## Claims

1. A composite membrane composed of a porous substrate and a porous separation layer which is composed of a zeolite of the MFI type, wherein the separation layer is produced by a hydrothermal synthesis in which the molar ratio of silicon to aluminum in the synthesis solution is greater than 120 and the substrate comprises less than 10% by weight of aluminum in elemental or chemically bound form in a zone of at least 100 nm adjacent to the separation layer, and wherein the molar ratio of silicon to aluminum in the separation layer is greater than 120 and the substrate material has an asymmetrical structure.

2. The composite membrane according to claim 1, wherein the substrate is a body having penetrating pores having a pore diameter of from 5 nm to 3 µm.

3. The composite membrane according to claim 1 or 2, wherein the substrate is selected from the group consisting of steels, sintered metals, oxide ceramic materials and metal oxides.

4. A process for the production of a composite membrane which comprises at least one porous substrate and at least one porous separation layer, the separation layer comprising at least one zeolite of the MFI type and the molar ratio of silicon to aluminum in the separation layer being greater than 120, and the substrate comprising less than 10% by weight of aluminum in elemental or chemically bonded form in a zone of at least 100nm adjacent to the separation layer, and the substrate material having an asymmetrical structure, comprising the following process steps:
(a) hydrothermal synthesis of a synthesis solution, in which the molar ratio of silicon to aluminum is greater than 120, on a substrate by bringing the substrate into contact with the synthesis solution over a period of from 1 to 100 hours at from 100 to 250°C,
(b) washing of the membrane resulting from process step (a) with water or an acidic solution for a period of from 5 to 120 minutes,
(c) drying of the membrane at from 5 to 40°C over a period of from 1 to 100 hours in the presence of a flowing or stationary gas,
(d) calcination of the membrane at a heating rate of from 0.1 to 1 K/min up to a temperature of from 200 to 600°C, residence being effected at the final temperature for from 30 to 500 minutes and then cooling being effected at a rate of 0.1 K/min to 10 K/min.

5. The process according to claim 4, wherein the substrate material used in process step (a) is subjected, before the hydrothermal synthesis, to a seeding step in which a layer of seed particles which at least partly covers that side of the substrate which is to be coated is applied to said side.

6. The process according to claim 4 or 5, wherein, in the hydrothermal synthesis of the synthesis solution on the substrate, access of the synthesis solution to the surface not to be coated or to the surfaces of the substrate which are not coated is substantially prevented by a medium which is present in the pores of the substrate and on the surface not to be coated or the surfaces not to be coated.

7. A process for separating olefin-comprising mixtures in at least one membrane apparatus, comprising at least one membrane, in which the olefin-comprising mixture flows into the membrane apparatus, is brought into contact with the at least one membrane and is separated into a stream passing through the membrane and a stream not passing through the membrane, wherein a composite membrane according to any of claims 1 to 3 or which can be obtained by the process according to any of claims 4 to 6 is used in the process.

8. The process according to claim 7, wherein the separation is carried out at from 20 to 200°C.

9. The process according to claim 7 or 8, wherein, in the separation of the olefin-comprising mixture, the pressure on the permeate side of the membrane is from 0.01 to 10 bar (abs) and/or the pressure on the retentate side of the membrane is higher than on the permeate side.

10. The use of the composite membrane according to any of claims 1 to 3 or which can be obtained by the process according to any of claims 4 to 6 for separating olefin-comprising mixtures.

11. The use according to claim 10, wherein the mixture comprises isomeric butenes.

## Revendications

1. Membrane composite, constituée d'un support poreux et d'une couche de séparation poreuse, qui est constitué d'un zéolithe de type MFI, **caractérisée en ce que** la couche de séparation est réalisée par synthèse hydrothermale, par laquelle le rapport molaire silicium/aluminium dans la solution de synthèse est supérieur à 120 et le support contient dans une zone adjacente à la couche de séparation d'au moins 100 nm moins de 10 % en poids d'aluminium sous forme élémentaire ou chimiquement liée et **en ce que** le rapport molaire silicium/aluminium dans la couche de séparation est supérieur à 120 et le matériau de support est construit de manière asymétrique.

2. Membrane composite selon la revendication 1, **caractérisée en ce que** le support est un corps comportant des pores de part en part d'un diamètre de pores de 5 nm à 3 µm.

3. Membrane composite selon la revendication 1 ou 2, **caractérisée en ce que** le support est choisi parmi le groupe constitué des aciers, des métaux frittés, des matériaux céramiques d'oxyde et des oxydes de métal.

4. Procédé de réalisation d'une membrane composite, comprenant au moins un support poreux et au moins une couche de séparation poreuse, la couche de séparation contenant au moins un zéolithe de type MFI et le rapport molaire silicium/aluminium dans la couche de séparation étant supérieur à 120 et le support contenant dans une zone adjacente à la couche de séparation d'au moins 100 nm moins de 10 % en poids d'aluminium sous forme élémentaire ou chimiquement liée et le matériau de support étant construit de manière asymétrique, **caractérisé en ce qu'**il comprend les étapes suivantes :
(a) synthèse hydrothermale d'une solution de synthèse, dans laquelle le rapport molaire silicium/aluminium est supérieur à 120, sur un support, en faisant entrer en contact le support avec la solution de synthèse sur une durée de 1 à 100 h, à une température de 100 à 250°C,
(b) rinçage de la membrane obtenue dans l'étape (a) du procédé à l'eau ou avec une solution acide pendant une durée de 5 à 120 minutes,
(c) séchage de la membrane à une température de 5 à 40°C pendant une durée de 1 à 100 h en présence d'un gaz affluant ou au repos,
(d) calcination de la membrane à une vitesse de chauffage de 0,1 à 1 K/min jusqu'à une température de 200 à 600°C, la température finale étant maintenue de 30 à 500 minutes et étant ensuite refroidie à une vitesse de 0,1 k/min à 10 K/min.

5. Procédé selon la revendication 4, **caractérisée en ce que** le matériau de support utilisé dans l'étape (a) du procédé subit avant la synthèse hydrothermale une étape d'amorçage, dans laquelle sur le côté à recouvrir du support est disposée une couche de particules d'amorçage recouvrant au moins en partie ce côte.

6. Procédé selon la revendication 4 ou 5, **caractérisé en ce que** lors de la synthèse hydrothermale de la solution de synthèse sur le support, l'accès de la solution de synthèse à la surface qui n'est pas à recouvrir ou aux surfaces qui ne sont pas à recouvrir du support est essentiellement empêché par un milieu qui se trouve dans les pores du support et sur la surface qui n'est pas à recouvrir ou les surfaces qui ne sont pas à recouvrir.

7. Procédé de séparation de mélanges contenant des oléfines dans au moins un dispositif de membrane, contenant au moins une membrane, dans lequel le mélange contenant de s oléfines afflue dans le dispositif à membrane, avec lequel au moins une membrane entre en contact et qui est séparé en un flux passant par la membrane et un flux ne passant pas par la membrane, **caractérisé en ce que** le procédé utilise une membrane composite selon l'une quelconque des revendications 1 à 3 ou pouvant être obtenue par le procédé selon l'une quelconque des revendications 4 à 6.

8. Procédé selon la revendication 7, **caractérisé en ce que** la séparation est effectuée à une température de 20 à 200°C.

9. Procédé selon la revendication 7 ou 8, **caractérisé en ce que** lors de la séparation du mélange contenant des oléfines, la pression sur le côté du perméat de la membrane est de 0,01 à 10 bar (a) et/ou la pression sur le côté du rétentat de la membrane est plus élevée que sur le côté du perméat.

10. Utilisation de la membrane composite selon l'une quelconque des revendications 1 à 3 ou pouvant être obtenue par le procédé selon l'une quelconque des revendications 4 à 6 pour la séparation de mélanges contenant des oléfines.

11. Utilisation selon la revendication 10, **caractérisée en ce que** le mélange contient du butène isomère.
